# EUROPÄISCHE PATENTSCHRIFT

(11) **EP 3 177 396 B1**
(45) Veröffentlichungstag und Bekanntmachung des Hinweises auf die Patenterteilung: **20.03.2019**
(21) Anmeldenummer: 15734598.4
(22) Anmeldetag: 24.06.2015
(51) Int. Cl.: B01J 20/22, B01J 20/30, B01J 20/32, B01J 31/16, C01B 37/00, C07F 15/02

(54) **VERFAHREN ZUR HERSTELLUNG EINES ADSORBENS AUS METALLORGANISCHEN GERÜSTSTRUKTUREN (MOF)**
METHOD FOR PRODUCING AN ADSORBENT FROM ORGANOMETALLIC FRAMEWORK STRUCTURES (MOF)
PROCÉDÉ SERVANT À FABRIQUER UN ADSORBANT À PARTIR DE STRUCTURES ORGANO-MÉTALLIQUES (MOF)

(30) Priorität: 06.08.2014 DE 102014215568
(43) Veröffentlichungstag der Anmeldung: 14.06.2017
(73) Patentinhaber: Fraunhofer Gesellschaft zur Förderung der angewandten Forschung E.V., 80686 München (DE)
(72) Erfinder: JEREMIAS, Felix, 79106 Freiburg (DE); HENNINGER, Stefan, 79346 Endingen (DE)
(86) Internationale Anmeldenummer: PCT/EP2015/064189
(87) Internationale Veröffentlichungsnummer: WO 2016/020104

(56) Entgegenhaltungen:
- WO-A1-2005/049484
- WO-A1-2007/014678
- WO-A1-2013/004726
- WO-A1-2014/012989
- WO-A2-2007/118841
- Felix Jeremias: "Synthesis and characterization of metal-organic frameworks for heat transformation applications", , 1. Juli 2014 (2014-07-01), Seiten 1-2, XP055217169, Gefunden im Internet: URL:http://publica.fraunhofer.de/dokumente /N-328221.html [gefunden am 2015-09-30] & Felix Jeremias: "Synthesis and Characterization of metal-organic Frameworks for Heat Transformation Applications", , 14. Juli 2014 (2014-07-14), Seiten 1-61, XP055217265, Düsseldorf Gefunden im Internet: URL:http://publica.fraunhofer.de/eprints/u rn_nbn_de_0011-n-3282213.pdf [gefunden am 2015-09-30]

## Beschreibung

Die vorliegende Erfindung betrifft ein Verfahren zur Herstellung eines Absorbens aus metallorganischen Gerüststrukturen (MOF), bei dem mindestens ein Metallsalz mit mindestens einem organischen Liganden umgesetzt wird. Die Umsetzung erfolgt bei einer Temperatur größer 100 °C in einem Lösungsmittelgemisch, das DMSO und Wasser enthält. Es wird außerdem ein mit dem erfindungsgemäßen Verfahren hergestelltes Absorbens bzw. ein mit einem solchen Absorbens beschichtetes Substrat sowie Verwendungsmöglichkeiten eines solchen Absorbens bzw. Substrates beschrieben.

Metallorganische Netzwerkverbindung *(metal-arganic frameworks,* MOFs) zählen zu den mikroporösen Materialien. Auf molekularer Ebene bestehen sie aus kationischen Metallionen Clustern *(Secondary Building Units,* SBUs), die durch organische, polyvalente koordinative Bindungen ausbildende Anionen *(Linker)* verbrückt sind. Aufgrund des hochsymmetrischen, kristallinen Aufbaus der Gitterstrukturen und der Tatsache, dass die Porosität bereits auf molekularer Ebene entsteht, zählen MOFs zu den Materialien mit der größten inneren Oberfläche (bis *S_{BET} >* 4000 m²/g) überhaupt, und aufgrund ihrer chemischen Vielfalt sind sie von großem Interesse für Anwendungen wie Gasspeicherung, Katalyse, Sorptionswärmetransformation, und viele mehr, wo sie Materialien wie Silicagele, Zeolithe oder Aktivkohlen ergänzen oder ersetzen können. Aufgrund der zahlreichen Kombinationsmöglichkeiten von SBUs und Clustern sind bereits viele Tausend Strukturen bekannt.

MOFs werden künstlich hergestellt, dazu gibt es unterschiedliche Möglichkeiten. Allen gemein ist, dass Metall-Kationen und Linker-Anionen zur Reaktion gebracht werden, und zwar so langsam und kontrolliert, dass sich die gewünschte kristalline Phase ausbilden kann. Von wenigen Ausnahmen abgesehen (anodische Synthese oder galvanische Verdrängung) werden dabei die Metallkationen (in Form eines leicht löslichen Metallsalzes) in hoher Konzentration vorgelegt. Die Anionen werden in geringer ·Konzentration *in-situ* aus der korrespondierenden freien Säure (ebenfalls vorgelegt) durch Deprotonierung erzeugt, um eine langsame, kontrollierte Kristallbildung zu erzielen.

Die Deprotonierung kann durch mehrere Methoden erzielt werden, etwa durch Zugabe einer basischen Verbindung, kathodisch durch Reduktion, oder, klassisch, durch Solvothermalsynthesen, wobei die Deprotonierung durch Erhitzen erfolgt (Gleichgewichtsverschiebung oder Bildung basischer Zersetzungsprodukte des Lösungsmittels). Die meisten MOFs können bislang nur durch Solvothermalsynthese hergestellt werden.

Häufig im Stand der Technik verwendete Lösungsmittel sind, in Reinform oder als Gemisch, Dialkylformamide, niedere Alkohole und Wasser (dann: "Hydrothermalsynthese"). Je nach verwendetem MOF führen dabei nur wenige Lösungsmittel zur Bildung der gewünschten Struktur. Bei falscher Wahl des Lösungsmittels wird dann ein amorphes, unporöses oder auch gar kein Produkt erhalten. Zahlreiche MOFs, insbesondere hochporöse MIL-Strukturen, die als Sorptionsmaterial für Wärmetransformationsanwendungen eingesetzt werden sollen, können nach Stand der Technik nur unter Verwendung von Wasser als Lösungsmittel hergestellt werden.

In US 2010/0226991 A1 wird eine Hydrothermalsynthese bei 100 °C beschrieben. Die Reaktion könnte somit theoretisch unter Atmosphärendruck und in einer offenen Apparatur durchgeführt werden, auch wenn dazu in US 2010/0226991 A1 nicht angeregt wird. Als Reaktionsgemisch dient eine Suspension von Trimesinsäure in wässriger Lösung von FeCl₃ · 6 H₂O. Diese Synthese liefert MOFs mit geringer Kristallinität und innerer Oberfläche. Da in Suspension gearbeitet wird, werden die MOFs als Gemisch mit nicht umgesetzter Linker-Verbindung erhalten. Eine aufwendige Reinigung ist daher erforderlich. Zudem ist diese Synthese für Beschichtungsverfahren nicht geeignet, da Partikel der Linkerverbindung unkontrollierbar in die Schicht eingelagert werden, wodurch unerwünschte Materialeigenschaften entstehen.

Die US 2010/0226991 A1 beschreibt außerdem eine Hydrothermalsynthese bei 130 °C mit Zusatz von Flusssäure, wobei die Fe³⁺-Kationen in situ aus metallischem Eisen und Salpetersäure dargestellt werden. Diese Synthese muss allerdings unter Überdrück im Autoklaven durchgeführt werden.

In US 2009/0227446 A1 wird eine Hydrothermalsynthese mit einem ähnlichen Reaktionsgemisch unter Mikrowelleneinstrahlung bei 200 °C beschrieben. Auch diese muss unter Überdruck und in einem geschlossen Reaktionsgefäß durchgeführt werden.

Dokument WO 2007/118841 A2 offenbart ein Verfahren zur Herstellung eines porösen metallorganischen Gerüstmaterials den Schritt enthaltend, Umsetzung eines Reaktionsgemisches enthaltend mindestens eine Aluminium-Verbindung und Fumarsäure oder ein Salz davon in Gegenwart eines (nicht-wässrigen) organischen Lösemittels bei einer vorgegebenen Temperatur und einem vorgegebenen Druck. Als Lösungsmittel sind grundsätzlich alle Lösungsmittel oder alle Lösungsmittelgemische denkbar, z.B. DMSO. Der Begriff "nicht-wässrig" bezieht sich vorzugsweise auf ein Lösemittel, das einen Höchstwassergehalt von 10 Gew.-%, mehr bevorzugt 5 Gew.-%, weiterhin mehr bevorzugt 1 Gew.-%, weiterhin bevorzugt 0,1 Gew.-%, besonders bevorzugt 0,01 Gew.-% bezogen auf das Gesamtgewicht des Lösemittels nicht überschreitet.

Insgesamt betrachtet weisen die im Stand der Technik beschriebenen Synthesemethoden folgende Nachteile auf:
- Aufgrund der typischen Reaktionstemperaturen (>100 °C) muss unter Überdruck und in entsprechenden Gefäßen (Autoklaven) gearbeitet werden. Dies erschwert die Reaktionskontrolle (kein Einblick in die Gefäße) und erhöht den Kostenaufwand enorm, insbesondere dann, wenn modifizierte Varianten der Solvothermalsynthese verwendet werden sollen (z.B. Beschichtungen durch Temperaturgradienten oder kathodische Deprotonierung). Eine kontinuierliche Syntheseführung, welche aus Gründen der Prozessökonomie wünschenswert ist, ist bei Synthesen unter Überdruck besonders schwierig zu verwirklichen.
- Die als Linker verwendeten Verbindungen, typischerweise aromatische oder olefinische Di-, Tri- oder Tetracarbonsäuren, sind meist schwer wasserlöslich und lösen sich erst bei Temperaturen > 100 °C im Reaktionsmedium auf. Beim Abkühlen fallen sie wieder aus. Das nach Abschluss der Reaktion isolierte MOF ist daher i.d.R. mit nicht umgesetzten Resten des Linkermoleküls verunreinigt, die durch aufwändiges Waschen mit organischen Lösungsmitteln entfernt werden müssen. Eine einfache Abtrennung des MOFs aus der Syntheselösung (z.B. durch Filtration) ist ebenfalls nicht möglich, was einer kontinuierlichen Reaktionsführung ebenfalls im Wege steht.
- Die aus dem Stand der Technik bekannten Hydrothermalsynthesen für MOFs bei *T* ≤ 100°C liefern MOFs mit vergleichsweise geringer Kristallinität und innerer Oberfläche, dazu in Form von sehr kleinen Partikeln, gar Nanopartikeln. Da hier in Suspension gearbeitet wird, werden auch diese MOFs als Gemisch mit nicht umgesetzter Linker-Verbindung erhalten. Eine aufwändige Reinigung ist daher erforderlich. Für Beschichtungsverfahren sind die entsprechenden Synthesen nicht geeignet, weil Partikel der Linker-Verbindung unkontrollierbar in die Schicht eingelagert werden, wodurch unerwünschte Materialeigenschaften entstehen.

Ausgehend vom Stand der Technik ist es demnach die Aufgabe der vorliegenden Erfindung, ein Verfahren zur Herstellung eines Absorbens aus metallorganischen Gerüststrukturen bereitzustellen, welches bei Atmossphärendruck und aus homogener Lösung erfolgen kann und durch welches die Schwierigkeiten der im Stand der Technik beschriebenen Verfahren überwunden werden.

Diese Aufgabe wird durch ein Verfahren mit den Merkmalen des Patentanspruches 1 gelöst. Die abhängigen Patentansprüche stellen vorteilhafte Weiterbildungen dar. Erfindungsgemäß wird somit ein Verfahren zur Herstellung eines Absorbens aus metallorganischen Gerüststrukturen (MOF) bereitgestellt, bei dem mindestens ein Metallsalz mit mindestens einem organischen Liganden umgesetzt wird. Die Umsetzung erfolgt dabei bei einer Temperatur größer 100 °C in einem Lösungsmittelgemisch, das zwischen 50 und 99 Gew.-% DMSO und zwischen 1 und 50 Gew.-% Wasser enthält, wobei die Summe der gewichtprozentualen Anteile von DMSO und Wasser im Lösungsmittelgemisch 100 Gew.-% beträgt.

Das erfindungsgemäße Verfahren zeichnet sich dadurch aus, dass als Lösungsmittel anstelle von Wasser ein zeotropes DMSO-Wasser-Gemisch verwendet wird. DMSO ist dabei kein übliches in der MOF-Synthese oder allgemein der Synthese anorganischer Festkörper eingesetztes Lösungsmittel. Durch die Verwendung eines DMSO und Wasser enthaltenden Lösungsmittelgemisches kann auch bei Atmosphärendruck ein Siedepunkt des Reaktionsgemisches von über 100 °C erreicht werden. Es ist somit durch das erfindungsgemäße Verfahren möglich, ein klassisch hydrothermal erzeugtes MOF bei Atmosphärendruck herzustellen. Dadurch werden Kosten eingespart, da überdruckfeste Apparaturen nicht erforderlich sind. Weiterhin ist das verwendete DMSO und Wasser enthaltende Lösungsmittelgemisch ein hervorragendes Lösungsmittel für de meisten Metallsalze und organischen Verbindungen, wodurch das Reaktionsgemisch in Form einer homogenen Lösung vorliegt. Dadurch ist die Realisierung einer großtechnischen, auch kontinuierlichen Produktion erheblich einfacher, weil entstandenes MOF durch Filtration aus dem laufenden Prozess entfernt werden kann, da es sich dabei schließlich um den einzigen in der Lösung suspendierten Feststoff handelt.

Dadurch, dass das Verfahren bei Atmosphärendruck und aus homogener Lösung durchgeführt werden kann ist somit eine kontinuierliche Syntheseführung möglich, wodurch das erfindungsgemäße Verfahren deutlich ökonomischer als die aus dem Stand der Technik bekannten Verfahren durchgeführt werden kann.

Die Verwendung von Wasser im erfindungsgemäßen Verfahren ist notwendig, da die herzustellenden MOFs in der Regel auch im dehydratisierten Zustand Wassermoleküle enthalten. Es muss also zumindest ein gewisser Anteil Wasser im Reaktionsgemisch vorhanden sein.

Mit dem erfindungsgemäßen Verfahren können MOFs mit hoher Kristallinität und großer innerer Oberfläche erhalten werden. Die Verwendung eines DMSO und Wasser enthaltenden Lösungsmittelgemisches ist hierbei entscheidend für den Erfolg einer unter Normaldruck und aus homogener Lösung erfolgenden MOF-Synthese. So können bei Verwendung anderer Lösungsmittel anstelle von DMSO im Lösungsmittelgemisch entweder lediglich röntgenamorphe, unporöse Strukturen erhalten werden oder die Lösung geliert sofort bzw. es wird überhaupt kein Feststoff erhalten. Durch die Verwendung eines DMSO und Wasser enthaltenden Lösungsmittelgemisches im erfindungsgemäßen Verfahren können also MOFs mit hoher Kristallinität und großer innerer Oberfläche erhalten werden, wobei die Synthese dabei gleichzeitig unter Atmosphärendruck und aus homogener Lösung durchgeführt werden kann.

In einer bevorzugten Variante des erfindungsgemäßen Verfahrens enthält das Metallsalz mindestens ein chemisch reduzierbares Anion. Ein solches chemisch reduzierbares Anion wird durch das im Reaktionsgemisch enthaltene DMSO sowie dessen Zersetzungsprodukte chemisch reduziert. Das dabei entstehende reduzierte Produkt unterstützt die Deprotonierung des organischen Liganden und damit die Bildung des MOF.

Das chemisch reduzierbare Anion ist bevorzugt ausgewählt aus der Gruppe bestehend aus Nitrat, Chlorit, Chlorat, Perchlorat, Bromit, Bromate, Perbromat, lodit, lodat, Periodat, Sulfat, Hydrogensulfat oder Mischungen hiervon.

In einer besonders bevorzugten Variante des erfindungsgemäßen Verfahrens ist das Metallsalz ein Metallnitrat. Handelt es sich bei dem verwendeten Metallsalz um ein Metallnitrat so wird bei der Umsetzung das Nitrat durch DMSO sowie dessen Zersetzungsprodukte zu Nitrit reduziert, welches basischer ist und so die Deprotonierung des organischen Liganden und damit die Bildung des MOF unterstützt.

DMSO und Metallnitrate gelten gemäß ihrer Sicherheitsdatenblätter als nicht kompatible Chemikalien. Dies ist offenbar darauf zurückzuführen, dass sich relativ leicht schwerlösliches DMSO-solvatisiertes Metallnitrat bilden kann, was potentiell explosive Wirkung besitzt. Durch den Wasseranteil im Reaktionsgemisch sowie die erhöhten Temperaturen des erfindungsgemäßen Verfahrens wird das Risiko einer Explosion verringert. Weitere Möglichkeiten zur Minimierung des Risikos einer Explosion können Ausführungsbeispiel 1 entnommen werden.

Das Metall ist ausgewählt aus der Gruppe bestehend aus Fe, Zn, Cu, Co, Ru, Os, Mn, Ni und Seltenerdmetallen.

In einer weiteren bevorzugten Variante des erfindungsgemäßen Verfahrens weisen die organischen Liganden verbrückende Sauerstoff-, Stickstoff oder Schwefel-Atome auf.

Weiterhin ist bevorzugt, dass die organischen Liganden ausgewählt sind aus der Gruppe bestehend aus zweizähnigen Liganden, mehrzähnigen Liganden und Mischungen hiervon.

Eine weitere bevorzugte Variante des erfindungsgemäßen Verfahrens sieht vor, dass die organischen Liganden ausgewählt sind aus der Gruppe bestehend aus Dicarbonsäuren, Tricarbonsäuren, Imidazolen, Triazolen und Mischungen hiervon.

Weiterhin ist bevorzugt, dass die organischen Liganden ausgewählt sind aus der Gruppe bestehend aus Trimesinsäure, Terephthalsäure, 4,4'-Bipyridin, Biphenylbisulfonsäure, 2,6-Naphthalindicarbonsäure, Fumarsäure, Isophthalsäure, Phtahlsäure, Oxalsäure und Mischungen hiervon.

Eine weitere bevorzugte Variante des erfindungsgemäßen Verfahrens sieht vor, dass das Lösungsmittelgemisch zwischen 10 und 30 Gew.-%, besonders bevorzugt zwischen 15 und 25 Gew.-%, Wasser enthält und/oder zwischen 70 und 90 Gew.-%, besonders bevorzugt zwischen 75 und 85 Gew.-%, DMSO enthält. Hierbei beträgt die Summe der gewichtsprozentualen Anteile von DMSO und Wasser im Lösungsmittelgemisch 100 Gew.-%. Der prozentuale Anteil an DMSO und Wasser im Lösungsmittelgemisch hat Einfluss auf den Siedepunkt des Lösungsmittelgemisches. Der gewünschte Siedepunkt des Lösungsmittelgemisches kann so durch Wahl der entsprechenden Anteile an DMSO und Wasser im Lösungsmittelgemisch eingestellt werden. Beispielsweise hat das Lösungsmittelgemisch bei einem Anteil von 20 Gew.-% Wasser und 80 Gew.-% DMSO bei Atmosphärendruck einen Siedepunkt von 130 °C. Bis zu dieser Temperatur kann dieses Lösungsmittelgemisch folglich im flüssigen Zustand bei Atmosphärendruck erhitzt werden.

In einer weiteren bevorzugten Variante des erfindungsgemäßen Verfahrens erfolgt die Umsetzung unter Rückfluss und/oder bei einer Temperatur von 110 bis 180 °C, bevorzugt von 120 bis 150 °C, besonders bevorzugt von 125 bis 135 °C.

Weiterhin ist bevorzugt, dass die Umsetzung bei einem Druck von 1 bis 5 bar, bevorzugt bei Atmosphärendruck, durchgeführt wird. Besonders bevorzugt wird das erfindungsgemäße Verfahren ohne zusätzliche Druckbeaufschlagung durchgeführt.

Eine weitere bevorzugte Variante des erfindungsgemäßen Verfahrens sieht vor, dass das Reaktionsgemisch mindestens einen Zusatzstoff enthält, der ausgewählt ist aus der Gruppe bestehend aus Salpetersäure, Flusssäure, Salzsäure, Bromwasserstoffsäure, Methansulfonsäure, Toluolsulfonsäure, Sulfaminsäure oder Schwefelsäure und Mischungen hiervon. Durch die Anwesenheit solcher Zusatzstoffe im Reaktionsgemisch kann die Kristallinität der erhaltenen MOFs verbessert werden.

In einer weiteren bevorzugten Variante des erfindungsgemäßen Verfahrens beträgt die Reaktionsdauer 12 bis 48 h, bevorzugt 18 bis 30 h, besonders bevorzugt 22 bis 26 h.

Eine weitere bevorzugte Variante des erfindungsgemäßen Verfahrens sieht vor, dass das entstandene Adsorbens gewaschen wird. Das Waschen erfolgt dabei bevorzugt mit einem Lösungsmittelgemisch, welches zwischen 1 und 50 Gew.-%, bevorzugt zwischen 10 und 30 Gew.-%, besonders bevorzugt zwischen 15 und 25 Gew.-%, Wasser enthält und/oder zwischen 50 und 99 Gew.-%, bevorzugt zwischen 70 und 90 Gew.-%, besonders bevorzugt zwischen 75 und 85 Gew.-%, DMSO enthält, wobei die Summe der gewichtsprozentualen Anteile von DMSO und Wasser im Lösungsmittelgemisch bevorzugt 100 Gew.-% beträgt.

Weiterhin ist bevorzugt, dass nach der Umsetzung das Reaktionsgemisch aufgearbeitet wird, wobei der während der Umsetzung des Reaktionsgemisches entstandene Feststoff von der überstehenden Lösung abgetrennt, bevorzugt abzentrifugiert oder abfiltriert, wird.

Eine weitere bevorzugte Variante des erfindungsgemäßen Verfahrens sieht vor, dass mindestens ein mit dem Adsorbens zu beschichtendes Substrat als Kathode zusammen mit einer Gegenanode in das Reaktionsgemisch getaucht und anschließend die Umsetzung durchgeführt wird, wobei das Adsorbens in Form einer Schicht auf dem Substrat abgeschieden wird und während der Umsetzung eine Spannung zwischen den Elektroden angelegt ist, die zu Beginn der Umsetzung bevorzugt eine Stromdichte von 200-1000 mA/dm², besonders bevorzugt 300 bis 500 mA/dm², bewirkt.

Durch eine solche Kombination von kathodischer Beschichtung und Thermal-Gradient-Verfahren ist die Bildung einer funktionellen Schicht auf Substraten möglich, und zwar mit einer hohen Geschwindigkeit. Der geschwindigkeitsbestimmende Deprotonierungs-Vorgang des Thermal-Gradient-Verfahrens kann auf diese Weise nämlich elektrochemisch beschleunigt werden. An der Kathode finden hierbei beispielsweise folgende Reaktionen statt:

H₂O + e⁻ → ½ H₂ + OH⁻

H₂O + NO₃⁻ + 2e⁻ → NO₂⁻ + 2 OH⁻

Die entstehenden Hydroxylanionen können die organischen Liganden deprotonieren oder auch direkt in das MOF eingebaut werden, wodurch schnell kristalline Schichten erzeugt werden können.

Auch im Hinblick auf einen kontinuierlichen Prozess zur Produktion von freiem, ungeträgertem Adsorbens ist die eben beschriebene Variante des erfindungsgemäßen Verfahrens besonders interessant. So ist es möglich, dass das auf der Kathode gebildete MOF kontinuierlich abgestreift wird, und lediglich die aus der Reaktionsmischung entfernten Verbindungen nachdosiert werden müssen. So kann er eine sehr ökonomische Produktion erreicht werden.

Das verwendete Substrat ist bevorzugt elektrisch leitfähig. Besonders bevorzugt handelt es sich um ein Substrat aus elektrisch leitfähiger Keramik, elektrisch leitfähigem Kunststoff, Kupfer, Aluminium und/oder Stahl, ganz besonders bevorzugt aus rostfreiem Stahl der Typen 1.4301 und/oder 1.4401.

Es ist weiterhin bevorzugt, dass vor der Beschichtung das Reaktionsgemisch ohne Anlegen einer Spannung erwärmt wird, wobei diese Erwärmung bevorzugt für 20 bis 90 min, besonders bevorzugt für 40 bis 50 min, erfolgt.

Insbesondere weist die Schicht eine Schichtdicke von 120 bis 180 µm, bevorzugt 140 bis 160 µm, auf.

Ein mit dem erfindungsgemäßen Verfahren hergestelltes Adsorbens oder ein mit einem Adsorbens beschichtetes Substratunterscheidet sich dadurch von im Stand der Technik hergestellten Produkten, dass es Spuren von DMSO und Spuren von Zersetzungsprodukten von DMSO aufweist, welche aus dem Syntheseverfahren stammen. Solche Zersetzungsprodukte sind beispielsweise Methanthiol, Formaldehyd, Dimethylsulfid oder Dimethylsulfon.

Ein Adsorbens oder ein mit einem Adsorbens beschichtetes Substrat, welche mit dem erfindungsgemäßen Verfahren herstellbar ist, kann zur Gastrennung, Gasspeicherung, Katalyse oder sorptionsbasierten Wärmetransformation verwendet werden.

Die vorliegende Erfindung wird anhand der nachfolgenden Beispiele näher erläutert ohne die Erfindung auf die speziell dargestellten Parameter zu beschränken.

### Ausführungsbeispiel 1

Eine Lösung von 21,25 mmol (4,48 g) Trimesinsäure in 160 ml Dimethylsulfoxid wurde im 250-ml-Dreihalskolben auf 90 °C erwärmt, dann wurde unter Rühren eine zimmerwarme Lösung von 32 mmol (12,96 g) Fe(NO₃)₃ · 9 H₂O in 32 ml H₂O auf einmal zugegeben. Die gelbgrüne Lösung wurde für eine Dauer von 24 h unter Rühren refluxiert (Innentemperatur: 131°C).

Der entstandene Feststoff wurde abzentrifugiert und die überstehende Lösung wurde noch heiß in 1l Wasser gekippt, um das Ausfällen von potentiell explosivem Fe(NO₃)₃ · 6 Me₂SO als Nebenprodukt zu vermeiden. Nach der Aufreinigung (Waschen mit DMF [90°C, 5 h], Ethanol [60 °C, über Nacht] und Wasser [90 °C, 5 h]) erhielt man 4,49 g eines braunroten Feststoffs. Die einzige kristalline Phase wurde mittels Röntgen-Pulverdiffraktometrie als MIL-100 bestimmt.

### Ausführungsbeispiel 2

Es wurde wie in Ausführungsbeispiel 1 vorgegangen, jedoch nur für eine Dauer von 45 min refluxiert. Die so erhaltene, braune und leicht trübe Flüssigkeit wurde in ein doppelwandiges Gefäß gegeben. Die äußere Wand wurde auf eine Temperatur von 45 °C thermostatisiert.

Dann wurden zwei Bleche aus rostfreiem Stahl 1.4301 (1.4016 oder Aluminium können auch verwendet werden) der Abmessungen 50 x 50 x 1,5 mm abgeschliffen, mit Aceton entfettet und auf einen speziell angefertigten Heizkörper montiert, der in jeweils 10 mm Abstand mit Gegenelektroden versehen wurde. Die blanke Oberfläche der Bleche betrug 15 cm² je Seite. Der Versuchsaufbau wurde in die gekühlte Stamm-Lösung eingetaucht und die Heizleistung so geregelt (typischerweise 210 W), dass die Temperatur kurz unter der Oberfläche der zu beschichtenden Bleche 135 °C betrug (bestimmt durch ein in eine Bohrung eingebrachtes Thermoelement). Dann wurde eine Spannung eingestellt, die zu Versuchsbeginn eine Stromstärke von 100 mA bewirkte (Stromdichte: 10 A/dm²). Nach 25 Minuten war die Stromstärke auf 50 mA abgefallen, der Versuch wurde beendet, die eingebrachten Bleche demontiert und für jeweils 24 h in DMF und Ethanol eingelegt. Die gebildete rotbraune Schicht wurde durch Pulverdiffraktometrie als MIL-100 bestimmt.

Die so erzeugte Schicht ist ca. 150 µm dick, in sich stabil, aber nur lose mit dem Substrat verbunden. Eine fest haftende Schicht kann erzielt werden, wenn als Substrat z.B. der rostfreie Stahl 1.4568 (Handelsname17-7 PH®) verwendet wird.

## Patentansprüche

1. Verfahren zur Herstellung eines Adsorbens aus metallorganischen Gerüststrukturen (MOF), bei dem mindestens ein Metallsalz mit mindestens einem organischen Liganden umgesetzt wird,
**dadurch gekennzeichnet, dass** die Umsetzung bei einer Temperatur größer 100 °C in einem Lösungsmittelgemisch erfolgt, das zwischen 50 und 99 Gew.-% DMSO und zwischen 1 und 50 Gew.-% Wasser enthält, wobei die Summe der gewichtsprozentualen Anteile von DMSO und Wasser im Lösungsmittelgemisch 100 Gew.-% beträgt, und wobei das Metall ausgewählt ist aus der Gruppe bestehend aus Fe, Zn, Cu, Co, Ru, Os, Mn, Ni und Seltenerdmetallen.

2. Verfahren nach dem vorhergehenden Anspruch, **dadurch gekennzeichnet, dass** das Metallsalz mindestens ein chemisch reduzierbares Anion enthält, wobei das chemisch reduzierbare Anion bevorzugt ausgewählt ist aus der Gruppe bestehend aus Nitrat, Chlorit, Chlorat, Perchlorat, Bromit, Bromate, Perbromat, Iodit, Iodat, Periodat, Sulfat, Hydrogensulfat oder Mischungen hiervon, und wobei das Metallsalz besonders bevorzugt ein Metallnitrat ist.

3. Verfahren nach einem der vorhergehenden Ansprüche, **dadurch gekennzeichnet, dass** die organischen Liganden verbrückende Sauerstoff-, Stickstoff oder Schwefel-Atome aufweisen und/oder die organischen Liganden ausgewählt sind aus der Gruppe bestehend aus zweizähnigen Liganden, mehrzähnigen Liganden und Mischungen hiervon.

4. Verfahren nach einem der vorhergehenden Ansprüche, **dadurch gekennzeichnet, dass** die organischen Liganden ausgewählt ist aus der Gruppe bestehend aus Dicarbonsäuren, Tricarbonsäuren, Imidazolen, Triazolen und Mischungen hiervon und/oder die organischen Liganden ausgewählt sind aus der Gruppe bestehend aus Trimesinsäure, Terephthalsäure, 4,4'-Bipyridin, Biphenylbisulfonsäure, 2,6-Naphthalindicarbonsäure, Fumarsäure, Isophthalsäure, Phtahlsäure, Oxalsäure und Mischungen hiervon.

5. Verfahren nach einem der beiden vorhergehenden Ansprüche, **dadurch gekennzeichnet, dass** das Lösungsmittelgemisch zwischen 10 und 30 Gew.-%, besonders bevorzugt zwischen 15 und 25 Gew.-%, Wasser enthält und/oder zwischen 70 und 90 Gew.-%, besonders bevorzugt zwischen 75 und 85 Gew.-%, DMSO enthält, wobei die Summe der gewichtsprozentualen Anteile von DMSO und Wasser im Lösungsmittelgemisch 100 Gew.-% beträgt.

6. Verfahren nach einem der vorhergehenden Ansprüche, **dadurch gekennzeichnet, dass** die Umsetzung unter Rückfluss und/oder bei einer Temperatur von 110 bis 180 °C, bevorzugt von 120 bis 150 °C, besonders bevorzugt von 125 bis 135 °C, erfolgt.

7. Verfahren nach einem der vorhergehenden Ansprüche, **dadurch gekennzeichnet, dass** die Umsetzung bei einem Druck von 1 bis 5 bar, bevorzugt bei Atmosphärendruck, durchgeführt wird.

8. Verfahren nach einem der vorhergehenden Ansprüche, **dadurch gekennzeichnet, dass** das Reaktionsgemisch mindestens einen Zusatzstoff enthält, der ausgewählt ist aus der Gruppe bestehend aus Salpetersäure, Flusssäure, Salzsäure, Bromwasserstoffsäure, Methansulfonsäure, Toluolsulfonsäure, Sulfaminsäure oder Schwefelsäure und Mischungen hiervon.

9. Verfahren nach einem der vorhergehenden Ansprüche, **dadurch gekennzeichnet, dass** nach der Umsetzung das Reaktionsgemisch aufgearbeitet wird, wobei der während der Umsetzung des Reaktionsgemisches entstandene Feststoff von der überstehenden Lösung abgetrennt, bevorzugt abzentrifugiert oder abfiltriert, wird.

10. Verfahren nach einem der vorhergehenden Ansprüche **dadurch gekennzeichnet, dass** mindestens ein mit dem Adsorbens zu beschichtendes Substrat als Kathode zusammen mit einer Gegenanode in das Reaktionsgemisch getaucht und anschließend die Umsetzung durchgeführt wird, wobei das Adsorbens in Form einer Schicht auf dem Substrat abgeschieden wird und während der Umsetzung eine Spannung zwischen den Elektroden angelegt ist, die zu Beginn der Umsetzung bevorzugt eine Stromdichte von 200-1000 mA/dm², besonders bevorzugt 300 bis 500 mA/dm², bewirkt.

11. Verfahren nach dem vorhergehenden Ansprüche, **dadurch gekennzeichnet, dass** das Substrat ein Substrat aus elektrisch leitfähiger Keramik, elektrisch leitfähigem Kunststoff, Kupfer, Aluminium und/oder Stahl, bevorzugt aus rostfreiem Stahl der Typen 1.4301 und/oder 1.4401, ist.

12. Verfahren nach einem der beiden vorhergehenden Ansprüche, **dadurch gekennzeichnet, dass** die Schicht eine Schichtdicke von 120 bis 180 µm, bevorzugt 140 bis 160 µm, aufweist.

## Claims

1. Method for the production of an absorbent made of metal-organic framework structures (MOF), in the case of which at least one metal salt is converted with at least one organic ligand, **characterised in that** the conversion is effected at a temperature greater than 100°C in a solvent mixture which comprises between 50 and 99% by weight of DMSO and between 1 and 50% by weight of water, wherein the sum of the proportions by percentage weight of DMSO and water in the solvent mixture is 100% by weight, and wherein the metal is selected from the group consisting of Fe, Zn, Cu, Co, Ru, Os, Mn, Ni and rare earth metals.

2. Method according to the preceding claim, **characterised in that** the metal salt comprises at least one chemically reducible anion, the chemically reducible anion being selected preferably from the group consisting of nitrate, chlorite, chlorate, perchlorate, bromite, bromate, perbromate, iodite, iodate, periodate, sulphate, hydrogen sulphate or mixtures hereof, and the metal salt being particularly preferably a metal nitrate.

3. Method according to one of the preceding claims, **characterised in that** the organic ligands have bridging oxygen-, nitrogen or sulphur atoms and/or the organic ligands are selected from the group consisting of bidentate ligands, polydentate ligands and mixtures hereof.

4. Method according to one of the preceding claims, **characterised in that** the organic ligands are selected from the group consisting of dicarboxylic acids, tricarboxylic acids, imidazoles, triazoles and mixtures hereof, and/or the organic ligands are selected from the group consisting of trimesic acid, terephthalic acid, 4,4'-bipyridine, biphenylbisulphonic acid, 2,6-naphthalenedicarboxylic acid, fumaric acid, isophthalic acid, phthalic acid, oxalic acid and mixtures hereof.

5. Method according to one of the two preceding claims, **characterised in that** the solvent mixture comprises between 10 and 30% by weight, particularly preferably between 15 and 25% by weight, of water and/or between 70 and 90% by weight, particularly preferably between 75 and 85% by weight, of DMSO, the sum of the proportions by percentage weight of DMSO and water in the solvent mixture being 100% by weight.

6. Method according to one of the preceding claims, **characterised in that** the conversion is effected with reflux and/or at a temperature of 110 to 180ºC, preferably of 120 to 150ºC, particularly preferably of 125 to 135ºC.

7. Method according to one of the preceding claims, **characterised in that** the conversion is implemented at a pressure of 1 to 5 bar, preferably at atmospheric pressure.

8. Method according to one of the preceding claims, **characterised in that** the reaction mixture comprises at least one supplement, which is selected from the group consisting of nitric acid, hydrofluoric acid, hydrochloric acid, hydrobromic acid, methane sulphonic acid, toluene sulphonic acid, sulphamic acid or sulphuric acid and mixtures hereof.

9. Method according to one of the preceding claims, **characterised in that**, after conversion, the reaction mixture is reprocessed, the solid produced during the conversion of the reaction mixture being separated, preferably being centrifuged or filtered off, from the supernatant solution.

10. Method according to one of the preceding claims, **characterised in that** at least one substrate which is to be coated with the adsorbent is immersed in the reaction mixture as cathode together with a counter-anode and subsequently the conversion is implemented, the adsorbent being deposited on the substrate in the form of a layer, and, during conversion, a voltage being applied between the electrodes which effects a current density of 200 - 1,000 mA/dm², particularly preferably 300 to 500 mA/dm², at the beginning of the conversion.

11. Method according to one of the preceding claims, **characterised in that** the substrate is a substrate made of electrically conductive ceramic, electrically conductive plastic material, copper, aluminium and/or steel, preferably made of stainless steel of the types 1.4301 and/or 1.4401.

12. Method according to one of the two preceding claims, **characterised in that** the layer has a layer thickness of 120 to 180 µm, preferably 140 to 160 µm.

## Revendications

1. Procédé de fabrication d'un adsorbant constitué de réseaux organométalliques (MOF), dans lequel on fait réagir au moins un sel métallique avec au moins un ligand organique, **caractérisé en ce que** la réaction a lieu à une température supérieure à 100 °C dans un mélange de solvants qui contient de 50 à 99 % en poids de DMSO et de 1 à 50 % en poids d'eau, la somme des pourcentages en poids du DMSO et de l'eau dans le mélange de solvants étant de 100 % en poids, et le métal étant choisi dans le groupe consistant en Fe, Zn, Cu, Co, Ru, Os, Mn, Ni et les métaux des terres rares.

2. Procédé selon la revendication précédente, **caractérisé en ce que** le sel métallique contient au moins un anion chimiquement réductible, l'anion chimiquement réductible étant choisi de préférence dans le groupe consistant en les anions nitrate, chlorite, chlorate, perchlorate, bromite, bromates, perbromate, iodite, iodate, periodate, sulfate, hydrogénosulfate ou les mélanges de ceux-ci, et le sel métallique étant d'une manière particulièrement préférée un nitrate métallique.

3. Procédé selon l'une des revendications précédentes, **caractérisé en ce que** les ligands organiques comprennent des atomes d'oxygène, d'azote ou de soufre pontants, et/ou que les ligands organiques sont choisis dans le groupe consistant en les ligands bidentés, les ligands multidentés et les mélanges de ceux-ci.

4. Procédé selon l'une des revendications précédentes, **caractérisé en ce que** les ligands organiques sont choisis dans le groupe consistant en les acides dicarboxyliques, les acides tricarboxyliques, les imidazoles, les triazoles et les mélanges de ceux-ci, et/ou que les ligands organiques sont choisis dans le groupe consistant en l'acide trimésique, l'acide téréphtalique, la 4,4'-bipyridine, l'acide biphénylebisulfonique, l'acide 2,6-naphtalènedicarboxylique, l'acide fumarique, l'acide isophtalique, l'acide phtalique, l'acide oxalique et les mélanges de ceux-ci.

5. Procédé selon l'une des deux revendications précédentes, **caractérisé en ce que** le mélange de solvants contient de 10 à 30 % en poids, d'une manière particulièrement préférée de 15 à 25 %, en poids d'eau, et/ou de 70 à 90 % en poids, d'une manière particulièrement préférée de 75 à 85 % en poids, de DMSO, la somme des pourcentages en poids du DMSO et de l'eau dans le mélange de solvants étant de 100 % en poids.

6. Procédé selon l'une des revendications précédentes, **caractérisé en ce que** la réaction a lieu au reflux et/ou à une température de 110 à 180 °C, de préférence de 120 à 150 °C, d'une manière particulièrement préférée de 125 à 135 °C.

7. Procédé selon l'une des revendications précédentes, **caractérisé en ce que** la réaction est mise en oeuvre sous une pression de 1 à 5 bar, de préférence sous la pression atmosphérique.

8. Procédé selon l'une des revendications précédentes, **caractérisé en ce que** le mélange réactionnel contient au moins un additif qui est choisi dans le groupe consistant en l'acide nitrique, l'acide fluorhydrique, l'acide chlorhydrique, l'acide bromhydrique, l'acide méthanesulfonique, l'acide toluènesulfonique, l'acide sulfamique ou l'acide sulfurique, et les mélanges de ceux-ci.

9. Procédé selon l'une des revendications précédentes, **caractérisé en ce que**, après la réaction, le mélange réactionnel subit un traitement, le solide qui se forme pendant la réaction du mélange réactionnel étant séparé de la solution surnageante, de préférence par centrifugation ou par filtration.

10. Procédé selon l'une des revendications précédentes, **caractérisé en ce qu'**au moins un substrat devant être revêtu de l'adsorbant est, en tant que cathode et en même temps qu'une contre-anode, immergé dans le mélange réactionnel, puis que la réaction est mise en oeuvre, l'adsorbant se déposant sous forme d'une couche sur le substrat et, pendant la réaction, une tension étant appliquée entre les électrodes, qui, au début de la réaction, crée de préférence une densité de courant de 200 à 1000 mA/dm², d'une manière particulièrement préférée de 300 à 500 mA/dm².

11. Procédé selon la revendication précédente, **caractérisé en ce que** le substrat est un substrat constitué d'une céramique conductrice, d'un plastique conducteur, de cuivre, d'aluminium et/ou d'acier, de préférence d'acier inoxydable des types 1.4301 et/ou 1.4401.

12. Procédé selon l'une des deux revendications précédentes, **caractérisé en ce que** la couche présente une épaisseur de couche de 120 à 180 µm, de préférence de 140 à 160 µm.
